**Europäisches Patentamt**

**European Patent Office**

**Office européen des brevets**

(19)

(11) Veröffentlichungsnummer: **0 026 827**
**B1**

(12) **EUROPÄISCHE PATENTSCHRIFT**

(45) Veröffentlichungstag der Patentschrift:
**16.05.84**

(51) Int. Cl.³: **C 07 D 301/10,** B 01 J 23/96,
C 07 D 303/04

(21) Anmeldenummer: **80104899.2**

(22) Anmeldetag: **16.08.80**

(54) **Verfahren zur Herstellung von Ethylenoxid.**

(30) Priorität: **06.09.79 DE 2936036**

(43) Veröffentlichungstag der Anmeldung:
**15.04.81 Patentblatt 81/15**

(45) Bekanntmachung des Hinweises auf die Patenterteilung:
**16.05.84 Patentblatt 84/20**

(84) Benannte Vertragsstaaten:
**BE DE FR GB IT NL**

(56) Entgegenhaltungen:
**EP - A - 0 018 471**
**DE - A - 2 352 608**
**DE - A - 2 649 359**
**DE - A - 2 712 785**
**DE - A - 2 746 976**
**DE - B - 2 519 599**

**Die Akte enthält technische Angaben, die nach dem Eingang der Anmeldung eingereicht wurden und die nicht in dieser Patentschrift enthalten sind.**

(73) Patentinhaber: **EC ERDÖLCHEMIE GMBH,**
**Postfach 75 20 02, D-5000 Köln 71 (DE)**

(72) Erfinder: **Alter, Eduard, Dr., Narzissenweg 14,**
**D-6302 Lich 1 (Hessen) (DE)**
Erfinder: **Bruns, Ludwig, Dr., Am Kronenpützchen 26,**
**D-4047 Dormagen-Straberg (DE)**
Erfinder: **Volprecht, Werner, Sankt-Simeon-Strasse 8,**
**D-5151 Fliesteden (DE)**

(74) Vertreter: **Dill, Erwin, Dr., c/o BAYER AG Zentralbereich**
**Patente Marken und Lizenzen Bayerwerk,**
**D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von Ethylenoxid

Die Erfindung betrifft ein Verfahren zur Herstellung von Ethylenoxid durch Oxidation von Ethylen mit Sauerstoff in Gegenwart eines Silberkatalysators.

Es ist bekannt, zur Herstellung von Ehtylenoxid durch Oxidation von Ethylen mit Sauerstoff Katalysatoren einzusetzen, die Silber auf einem inerten Träger enthalten. Zur Erhöhung der partiellen Oxidation des Ethylens zu Ethylenoxid und zur gleichzeitigen Zurückdrängung der Totaloxidation des Ethylens zu Kohlendioxid und Wasser sind zahlreiche Verbesserungen dieses Verfahrens versucht worden. Beispielsweise beschreibt die DE-OS 23 00 512 die Herstellung von Silberkatalysatoren, die Kalium, Rubidium und/oder Cäsium enthalten, die gleichzeitig mit dem Silber auf den Träger gebracht werden. Weiterhin ist es bekannt, daß Silberkatalysatoren bei der Herstellung von Ethylenoxid im Laufe ihrer Betriebszeit in ihrer Aktivität und Selektivität nachlassen. Dieser Leistungsabfall verläuft kontinuierlich (DE-PS 10 67 421). Daher ist auch bereits vorgeschlagen worden, nach längerer Betriebszeit ermüdete Katalysatoren durch Behandlung mit alkoholischen Lösungen von Salzen der 1. Gruppe, vorzugsweise Salze des Cäsiums und Rubidiums zu reaktivieren (DE-AS 25 19 599).

Der Austausch des Katalysators bedeutet in großtechnischen Anlagen einen erheblichen Produktionsausfall und hohe Kosten. Auch die Reaktivierung kann nur während eines Stillstandes der Anlage durchgeführt werden. Die Dosierung der aufzubringenden Dotierungsstoffe ist dabei kritisch und schwierig und in hohem Maße vom jeweiligen Zustand des Katalysators abhängig.

In der älteren, nicht vorveröffentlichten EP-Patentanmeldung 18 471 ist ein Verfahren zum Aktivieren oder Reaktivieren von Silber-Träger-Katalysatoren zur Ethylenoxid-Herstellung beschrieben, bei dem das Einsatzgasgemisch durch eine mit Promotormetallverbindungen versehene Schicht leitet. Diese Schicht besteht aus einem üblichen Trägermaterial, das mit wäßrigen Lösungen von Barium- und/oder Alkalimetallsalzen vorher getränkt worden war. Die hierzu eingesetzten Salze haben jedoch bei der für die Ethylenoxid-Herstellung in Frage kommenden Temperatur eine so geringe Flüchtigkeit, daß der Transport nennenswerter Mengen der Promotorsalze auf diese Weise nicht vorstellbar ist.

In der DE-OS 23 52 608 wird die Ethylenoxid-Herstellung mit einem im Wirbelbett angeordneten Katalysator beschrieben, dem Chloride von Natrium, Kalium, Lithium, Barium oder Cadmium in Form von 0,1 bis 1 mm großen Kristallen pneumatisch mit Hilfe des Einsatzgasgemisches zugeführt werden können.

Es wurde nun ein Verfahren zur Herstellung von Ethylenoxid durch Oxidation von Ethylen mit Sauerstoff in Gegenwart eines Silberkatalysators bei einer Temperatur von 200 bis 300° C gefunden, wobei man dem Einsatzgasgemisch, das im wesentlichen aus Ethylen, molekularem Sauerstoff und gegebenenfalls inerten Gasen besteht, Alkalimetallverbindungen als Promotoren zusetzt, das dadurch gekennzeichnet ist, daß man mindestens eine solche Alkalimetallverbindung in Form eines aus einer wäßrigen Lösung eines oder mehrerer Alkalimetallsalze durch Verdüsen erzeugten Aerosols oder Nebels zusetzt oder das Einsatzgasgemisch durch eine oder über eine Salzschmelze leitet, die sich aus Kaliumformiat und Cäsiumacetat unterhalb von 300° C bildet.

Als Alkalimetall sei beispielsweise Lithium, Natrium, Kalium, Rubidium oder Cäsium, bevorzugt Kalium und/oder Cäsium, genannt.

Für das erfindungsgemäße Verfahren ist die Menge des eingebrachten Alkalimetallkations von größerer Bedeutung, wogegen die Art und Menge des gegebenenfalls mit eingebrachten Anions von geringerer Bedeutung ist. Als Menge des eingesetzten Alkalimetalls sei beispielsweise der Bereich von 1 ppb-Gramm Atom bis 1 ppm-Gramm Atom, berechnet als Alkalimetall und bezogen auf die Menge des Einsatzgasgemisches genannt. Bevorzugt sei eine Menge von 10 ppb-Gramm Atom bis 200 ppb-Gramm Atom genannt.

Als Alkalimetallverbindungen seien ihre Hydroxide, ihre anorganischen Salze, wie Carbonate, Hydrogencarbonate, Fluoride, Chloride, Bromide, Jodide, Nitrate, Sulfate, ihre organischen Salze, wie Formiate, Acetate, Oxalate, Tartrate oder Lactate, ihre Alkoholate, wie Methylate, Ethylate, tert.-Butylate oder Benzyl-Alkoholate, oder ihre Phenolate genannt.

Bevorzugt werden im erfindungsgemäßen Verfahren die Formiate und/oder Acetate von Lithium, Natrium, Kalium, Rubidium und/oder Cäsium zugesetzt. Diese genannten Salze können sowohl allein zugesetzt werden oder auch als Mischungen.

Besonders bevorzugt werden im erfindungsgemäßen Verfahren Mischungen von Kaliumformiat und Cäsiumacetat, deren Mischungsverhältnis so gewählt ist, daß sich eine Salzschmelze bereits unterhalb der im Prozeß erforderlichen Reaktionstemperatur, also unterhalb von beispielsweise 300° C, ausbildet. Bevorzugt sind Mischungen, deren Schmelzpunkte von 100 bis 300° C liegen. Besonders bevorzugt sind solche Mischungen, deren Schmelzpunkte von 100 bis 160° C liegen.

Der Anteil Kaliumformiat in einer solchen Mischung liegt im allgemeinen von 5 bis 90 Gewichtsprozent, bevorzugt von 20—50 Gewichtsprozent dieser Mischung.

Die Alkalimetallverbindungen werden dem Einsatzgasgemisch für die Herstellung von Ethylenoxid zugesetzt. Dieses Einsatzgasgemisch besteht im wesentlichen aus Ethylen, molekularem Sauerstoff und gegebenenfalls inerten Gasen. Falls der molekulare Sauerstoff in Form von Luft-Sauerstoff eingesetzt wird, sind die übrigen Bestandteile der Luft, wie Stickstoff, Kohlendioxid und Wasserdampf als

**0 026 827**

inerte Gase anzusehen.

Falls das Einsatzgasgemisch unter Verwendung von reinem Sauerstoff hergestellt wird, kann es zweckmäßig sein, ein Inertgas wie Stickstoff, Kohlendioxid, Äthan oder Methan, zuzusetzen, um den Sauerstoffgehalt in einem Bereich außerhalb der Explosionsgrenze zu halten.

Zur Beeinflussung des Reaktionsumfanges, d. h. des Umsatzes an Sauerstoff und Äthylen pro Reaktordurchgang und zur Unterdrückung der Kohlendioxid-Bildung können dem Einsatzgas, dem Stande der Technik gemäß, chlorierte Kohlenwasserstoffe, bevorzugt 1,2-Dichloräthan, als Inhibitor in einer Konzentration von etwa 0,5 bis 4 ppm, bezogen auf die Menge des Einsatzgasgemisches zugesetzt werden.

Die Zugabe der Alkalimetallverbindungen oder deren Mischungen zum Reaktoreinsatzgas kann nach dem erfindungsgemäßen Verfahren in verschiedener Weise erfolgen. Beispiele hierfür seien als Ausführungsform 1 und 2 beschrieben.

Bei der Ausführungsform 1 wird eine Mischung von Kaliumformiat und Cäsiumacetat in einer geeigneten apparativen Einrichtung als Schmelze vorgehalten. Über oder durch diese Schmelze wird ein Teil oder gegebenenfalls das gesamte Reaktoreinsatzgas geleitet, wobei es sich entsprechend dem Dampfdruck dieser Schmelze mit den genannten Verbindungen belädt. Der Beladungsgrad kann durch Änderung der Temperatur der Schmelze in weiten Grenzen beeinflußt werden. Statt des Reaktoreinsatzgases kann für den Transport der Alkalimetallverbindungen natürlich auch ein Inertgas, wie z. B. Stickstoff oder Methan, verwendet werden.

Die Ausführungsform 2 für das erfindungsgemäße Verfahren besteht darin, daß man die Alkalimetallverbindungen oder deren Mischungen als wäßrige Lösungen dem Reaktionsgas so zumischt, daß bei der Verwendung von Salzen oder deren Mischungen mit einem Schmelzpunkt oberhalb der Reaktionsgastemperatur Aerosole und bei einem Schmelzpunkt unterhalb der Gastemperatur Nebel entstehen.

Die Erzeugung der Aerosole oder Nebel kann z. B. durch Verdüsung der wäßrigen Lösung unter Benutzung eines Treibgases oder durch Verdampfungsentspannungsverdüsung erreicht werden.

Der erfindungsgemäße Zusatz von mindestens einer Alkalimetallverbindung kann dauernd oder zeitweise erfolgen. So kann beispielsweise dieser Zusatz unterbrochen werden, wenn der Katalysator die maximal erreichbare Selektivität zeigt und behält, wohingegen dieser Zusatz beim Absinken der Selektivität wieder aufgenommen werden kann.

Mit Hilfe des erfindungsgemäßen Verfahrens gelingt es, die bei der Ethylenoxidation gebräuchlichen Silberkatalysatoren während des laufenden Betriebes, also ohne daß eine Unterbrechung der Produktion erforderlich ist, so zu beeinflussen, daß eine optimale Reaktorausbeute erhalten bleibt.

Darüber hinaus kann das erfindungsgemäße Verfahren auch für eine Reaktivierung von solchen Katalysatoren vorteilhaft eingesetzt werden, die nach längerer Betriebszeit bereits einen Leistungsabfall zeigen.

Bei einer genügend langen Anwendung des erfindungsgemäßen Verfahrens, insbesondere in der Ausführungsform 2, kann die Leistung der desaktivierten Katalysatoren erheblich gesteigert und die zur Ethylenoxidbildung erforderliche Reaktionstemperatur beträchtlich gesenkt werden.

Beispiele

Die Beispiele und Vergleichsbeispiele wurden in einer Laborapparatur, bzw. Pilot-Anlage durchgeführt, die im folgenden beschrieben sind.

Der Labor-Versuchsreaktor besteht aus einem ölbeheizten Glasrohr mit Doppelmantel mit einer lichten Weite von 50 mm und einer Länge von 1000 mm, von der 100 mm mit einem inerten Trägermaterial gefüllt sind und als Vorheizzone dienen. Die Laborversuche wurden bei atmosphärischem Druck durchgeführt. Die analytische Überwachung des Reaktorproduktgasstromes erfolgt kontinuierlich durch einen Prozeß-Gaschromatographen. Die Laufzeit der Versuche betrug im Labor im Mittel 4 bis 5 Monate. Die Raum-Zeit-Geschwindigkeit beträgt: 250 Raumteile Gas pro Raumteil Katalysator und Stunde. Die Reaktionstemperaturen lagen zwischen 230° C und 242° C. Der Ethylen-Umsatz betrug bei den einzelnen Versuchen jeweils 9 bis 9,8%.

Die Pilot-Anlage besteht aus einem 10,80 m langen Rohr mit einer lichten Weite von 42 mm. Das Reaktionsrohr besteht aus einem Doppelmantel, in dessen äußeren Teil ein Wärmeträgermedium zirkuliert und die Reaktionstemperatur auf ±0,5° C konstant hält. Als Vorwärmestrecke sind 1,50 m eines inerten Katalysatorträgers eingebracht. Der Reaktionsdruck im Rohr beträgt 15 bar, die analytische Überwachung erfolgt kontinuierlich über einen Prozeß-Gaschromatograph. Die Raum-Zeit-Geschwindigkeit beträgt 3800 Raumteile Gas pro Raumteil Katalysator und Stunde.

Die Reaktionstemperaturen wurden so eingestellt, daß ein Ethylen-Umsatz von 9 bis 9,8% erreicht wurde.

3

Das für die Labor- und Pilot-Apparatur eingesetzte Gasgemisch besteht aus:

| | |
|---|---|
| $C_2H_4$ | 30 Vol.-% |
| $CH_4$ | 50 Vol.-% |
| $O_2$ | 8 Vol.-% |
| $CO_2$ | 4 Vol.-% |
| Inerte | 8 Vol.-% |

Dem eingesetzten Gasgemisch wurde als Inhibitor 1 bis 2 ppm 1,2-Dichloräthan zugesetzt.

Der für die Beispiele und Vergleichsbeispiele 1 bis 6 eingesetzte Katalysator ist ein Silber-Träger-Katalysator, bestehend aus 15 Gewichtsprozent Silber auf $\alpha$-Aluminiumoxid als Trägermaterial. Das Trägermaterial ist kugelförmig mit einem Durchmesser von 8 mm und einer spezifischen Oberfläche von 0,005 bis 0,05 m2/g. Für die Beispiele 1 bis 6 wurde nach DE-PS 12 11 607 frischgefälltes Silberoxid mit Milchsäure versetzt, bis eine klare Lösung entstanden war. In diese Lösung wurde der Katalysator-Träger eingebracht und das entstandene Gemenge wurde so lange unter vermindertem Druck erwärmt, bis die Lösung fast vollständig vom Träger aufgesaugt war. Der stark verklebte Träger wurde dann auf 250 bis 380° C erhitzt, wobei sich metallisches Silber bildete. Für das Beispiel 7 wurde ein nach DE-OS 23 00 512 hergestellter Katalysator mit 0,00081 g Äquivalent Cäsium pro kg Kontakt eingesetzt.

Für den Alkalimetallsalz-Zusatz hatte die Labor-Apparatur ein separat beheizbares Doppelmantelgefäß, das mit 5 g einer Salzmischung von Kaliumformiat und Cäsiumacetat im Molverhältnis 1 : 2 beschickt wurde. Die Salzmischung wurde auf 140° C aufgeheizt und lag ab 120° C flüssig vor. Über ein Rotameter wurden 10 Vol.-% des Einsatzgases mittels einer Glasfritte durch die flüssige Salzschmelze geleitet und dem Reaktoreinsatzgas wieder zudosiert.

Die Pilot-Anlage hatte ein beheizbares Doppelmantelgefäß von 3 l Volumen mit 100 g einer Salzmischung aus Kaliumformiat und Cäsiumacetat im Molverhältnis von 1 : 2. Das Gefäß wurde auf 140° C aufgeheizt. 50 l/h des Reaktoreinsatzgases wurden über eine Glasfritte durch die flüssige Schmelze geleitet und dem übrigen Reaktoreinsatzgas vor Eintritt in den Reaktor wieder zudosiert.

Tabelle 1 (Vergleichsbeispiele 1, 3 und 5 und Beispiele 2, 4, 6 und 7)

| Nr. | Reaktions-temperatur (°C) | Selektivität (%) | Äox-Konzentration (Vol.-%) | $C_2H_4$-Umsatz (%) | Laufzeit |
|---|---|---|---|---|---|
| 1 | 240 | 69,9 | 2,00 | 9,5 | 2 Monate |
| 2 | 235 | 77,0 | 2,24 | 9,7 | 2 Monate |
| 3 | 246 | 67,2 | 1,72 | 8,5 | 1 Monat |
| 4 | 242 | 76,9 | 2,18 | 9,4 | 1,5 Monate |
| 5 | 238 | 77,0 | 2,15 | 9,3 | |
| | 239 | 77,5 | 2,10 | 9,3 | nach 6 Monaten |
| 6 | 238 | 77,0 | 2,15 | 9,3 | |
| | 236 | 79,5 | 2,15 | 9,3 | nach 8 Monaten |
| 7 | 238 | 80,0 | 2,24 | 9,3 | 6 Monate |

## Vergleichsbeispiel 1

Ein in der Ethylenoxid-Produktion bereits eingesetzter Katalysator (Betriebszeit 6 Jahre; Herstellung wie weiter vorn beschrieben) wurde in der Labor-Apparatur ohne erfindungsgemäßen Zusatz von Alkalimetallsalz eingesetzt. Die gemessene Selektivität von 69,9% änderte sich im Laufe von 2 Monaten nicht.

## Beispiel 2

Ein bereits eingesetzter Katalysator analog Vergleichsbeispiel 1 wurde in der Labor-Apparatur eingesetzt und mit Hilfe der beschriebenen Einrichtung kontinuierlich mit einem mit 500 ppb Kaliumformiat und Cäsiumacetat beladenen Gasstrom bei der Oxidation von Ethylen beaufschlagt. Nach einer Laufzeit von 2 Montagen stieg die Selektivität von 69,9% auf 77,0% an und veränderte sich in den folgenden 3 Monaten nicht mehr.

## Vergleichsbeispiel 3

Ein analog Vergleichsbeispiel 1 bereits seit 6 Jahren in der Ethylenoxid-Produktion eingesetzter Katalysator wurde ohne Zusatz von Alkalimetallsalz in der Pilot-Apparatur eingesetzt. Der Katalysator wies nach einer Woche eine Selektivität von 67,2% bei einer Wärmeträgertemperatur von 246°C auf und änderte sich während der Versuchsdauer von 4 Wochen nicht.

## Beispiel 4

Ein analog Vergleichsbeispiel 3 bereits gebrauchter Katalysator wurde in der Pilot-Anlage eingesetzt und während der Ethylen-Oxidation mit einem mit 100 ppb Kaliumformiat und Cäsiumacetat beladenen Gasstrom beaufschlagt. Die Anfangsselektivität betrug 67% und stieg innerhalb von 9 Wochen auf 76,9% an und veränderte sich in den folgenden 6 Wochen nicht mehr.

## Vergleichsbeispiel 5

Ein neuer Katalysator, hergestellt nach DE-PS 12 11 607, wie oben beschrieben, wurde in der Pilot-Anlage eingesetzt, wobei dem Einsatzgas kein Alkalisalz zugesetzt wurde. Der Katalysator zeigt eine Anfangsselektivität von 77%, die im Laufe von 6 Monaten auf 75,5% abfiel. Dieser Abfall der Selektivität der Pilot-Anlage ist vergleichbar mit den Erfahrungen aus der großtechnischen Produktion.

## Beispiel 6

Der neu hergestellte Katalysator aus dem Vergleichsbeispiel 5 wurde in der Pilot-Anlage eingesetzt, wobei dem Einsatzgas die oben beschriebene Alkalisalzmenge zugesetzt wurde. Der Katalysator hatte eine Anfangsselektivität von 77%, die in den folgenden 8 Monaten sogar noch auf 79,5% gesteigert werden konnte.

## Beispiel 7

Ein nach DE-OS 23 00 512 hergestellter und weiter oben beschriebener Katalysator mit 0,00081 g-Äquivalent Cäsium pro kg Kontakt wurde in der Pilot-Anlage eingesetzt, wobei dem Einsatzgas Alkalimetallsalz zugesetzt wurde. Die Anfangsselektivität von 80% konnte über einen Zeitraum von 6 Monaten konstant gehalten werden. Ein gleicher Katalysator ohne den erfindungsgemäßen Zusatz ist nach 6 Monaten auf 75% Selektivität abgefallen.

## Beispiel 8

Die bereits beschriebene Labor-Apparatur wurde mit einem bereits in einer großtechnischen Anlage eingesetzten Kontakt (Betriebszeit 6 Jahre) gefüllt. In den Reaktoreinsatzgasstrom wurde über eine Zweistoffdüse eine wäßrige Alkalisalz-Lösung verdüst, die 0,04 molar an Cäsiumacetat und 0,04 molar an Kaliumformiat war. Als Treibmedium wurde das Reaktor-Einsatzgas benutzt. Die verdüste Flüssigkeitsmenge betrug etwa 1 ml/h. Die Eindüsung erfolgt solange, bis ein deutlich sichtbarer Anstieg der Selektivität zu verzeichnen war. Nach der Beendigung des Eindüsens stieg die Selektivität weiter bis auf ein Optimum von 79 bis 80%, das sich auch durch weiteres Eindüsen nicht mehr änderte. Die Versuchsergebnisse in Abhängigkeit von der Zeit sind in Tabelle 2 zusammengestellt.

Tabelle 2 (Beispiel 8)

Versuchsergebnisse für das Eindüsen einer wäßrigen Cäsium-/Kaliumsalz-Lösung

| Zeit Tage | Reaktions- temperatur °C | Selektivität % | Äox- Konzentration Vol.-% | Verdüste Flüssigkeits- menge ml/h |
|---|---|---|---|---|
| Start | 238 | 69,0 | 2,00 | 1 |
| 48 | 238 | 71,0 | 2,08 | 1 |
| 96 | 236 | 72,2 | 2,10 | 1 |
| 144 | 236 | 73,0 | 2,09 | 1 |

Tabelle 2 (Fortsetzung)

| Zeit Tage | Reaktions-temperatur °C | Selektivität % | Äox-Konzentration Vol.-% | Verdüste Flüssigkeits-menge ml/h |
|---|---|---|---|---|
| 192 | 235 | 74,3 | 2,12 | — |
| 240 | 233 | 75,6 | 2,10 | — |
| 288 | 231 | 76,9 | 2,15 | — |
| 336 | 231 | 77,8 | 2,13 | — |
| 384 | 231 | 78,4 | 2,16 | — |
| 432 | 231 | 79,6 | 2,15 | — |
| 480 | 231 | 79,6 | 2,13 | 1 |
| 528 | 231 | 79,6 | 2,15 | 1 |
| 576 | 231 | 79,7 | 2,13 | 1 |
| 624 | 231 | 79,6 | 2,13 | — |
| 960 | 231 | 79,6 | 2,14 | — |

Beispiel 9

Der Labor-Reaktor wurde mit einem Katalysator gefüllt, der bereits seit 6 Jahren in einer großtechnischen Anlage in Betrieb gewesen war. In die weiter oben beschriebene Vorwärmezone wurde an Stelle des inerten Katalysatorträgermaterials ein Material eingesetzt, das wie folgt hergestellt worden war:

$\alpha$-Aluminiumoxid mit einer spezifischen Oberfläche von 0,005 bis 0,05 m2/g wurde mit einer wäßrigen Lösung getränkt, die 2 molar an Cäsiumacetat und 2 molar an Kaliumformiat war. Nach dem Trocknen wurde dieser getränkte Katalysator-Träger in der Vorwärmezone auf 150°C aufgeheizt. Das Reaktor-Einsatzgas wurde vor dem Eintritt in die Vorwärmezone ebenfalls auf 150°C vorgewärmt. Die Selektivität konnte von anfangs 69,5% auf 76,7% nach 90 Tagen gesteigert werden. Weitere Einzelheiten zeigt die beigefügte Tabelle 3.

Tabelle 3 (Beispiel 9)

Versuchsergebnisse für das Einbringen eines festen Salzdepots im Reaktoreinsatzgasstrom

| Zeit Tage | Reaktions-temperatur °C | Selektivität % | Äox-Konzentration Vol.-% |
|---|---|---|---|
| Start | 242 | 69,5 | 2,05 |
| 15 | 242 | 70,8 | 2,07 |
| 25 | 242 | 71,5 | 2,05 |
| 40 | 242 | 72,5 | 2,00 |
| 50 | 241 | 73,4 | 2,05 |
| 60 | 240 | 74,4 | 2,08 |
| 70 | 240 | 75,3 | 2,05 |
| 80 | 239 | 76,1 | 2,00 |
| 90 | 239 | 76,7 | 2,08 |

6

**0 026 827**

**Patentansprüche**

1. Verfahren zur Herstellung von Ethylenoxid durch Oxidation von Ethylen mit Sauerstoff in Gegenwart eines Silberkatalysators bei einer Temperatur von 200 bis 300°C, wobei man dem Einsatzgasgemisch, das im wesentlichen aus Ethylen, molekularem Sauerstoff und gegebenenfalls inerten Gasen besteht; Alkalimetallverbindungen als Promotoren zusetzt, dadurch gekennzeichnet, daß man mindestens eine solche Alkalimetallverbindung in Form eines aus einer wäßrigen Lösung eines oder mehrerer Alkalimetallsalze durch Verdüsen erzeugten Aerosols oder Nebels zusetzt oder das Einsatzgasgemisch durch eine oder über eine Salzschmelze leitet, die sich aus Kaliumformiat und Cäsiumacetat unterhalb von 300°C bildet.

2. Verfahren nach Anspruch 1, dadurch gekennzeichnet, daß eine Mischung aus Kaliumformiat und Cäsiumacetat mit einem Anteil an Kaliumformiat von 5 bis 90 Gew.-% des Gesamtgewichts der Mischung eingesetzt wird.

**Claims**

1. Process for the preparation of ethylene oxide by oxidising ethylene with oxygen in the presence of a silver catalyst at a temperature of 200 to 300°C, alkali metal compounds being added as promoters to the feed gas mixture, which essentially consists of ethylene, molecular oxygen and, optionally, inert gases, characterised in that at least one such alkali metal compound is added in the form of an aerosol or mist, produced by atomising an aqueous solution of one or more alkali metal salts, or the feed gas mixture is passed through or over a salt melt formed from potassium formate and cesium acetate below 300°C.

2. Process according to Claim 1, characterised in that a mixture of potassium formate and cesium acetate with a potassium formate content of 5 to 90% by weight of the total weight of the mixture is employed.

**Revendications**

1. Procédé de production d'oxyde d'éthylène par oxydation d'éthylène avec de l'oxygène en présence d'un catalyseur à l'argent à une température de 200 à 300°C, dans lequel on ajoute des composés de métaux alcalins comme promoteurs au mélange gazeux constituant la charge, qui est principalement formé d'éthylène, d'oxygène moléculaire et, le cas échéant, de gaz inertes, caractérisé en ce qu'on ajoute au moins un tel composé de métal alcalin sous la forme d'un aérosol ou d'un brouillard produit par atomisation à partir d'une solution aqueuse d'un ou plusieurs sels de métal alcalin, ou bien on fait passer le mélange gazeux chargé à travers ou au-dessous d'une masse fondue de sel qui se forme au-dessous de 300°C à partir de formiate de potassium et d'acétate de césium.

2. Procédé suivant la revendication 1, caractérisé en ce qu'on utilise un mélange de formiate de potassium et d'acétate de césium contenant une proportion de formiate de potassium de 5 à 90% en poids par rapport au poids total du mélange.